Europäisches Patentamt

European Patent Office

Office européen des brevets

(11) Numéro de publication: **0 465 380 B1**

# FASCICULE DE BREVET EUROPEEN

(45) Date de publication du fascicule du brevet: **23.08.95**

(51) Int. Cl.6: **A61L 33/00**, B01D 67/00, B01D 71/42

(21) Numéro de dépôt: **91420199.1**

(22) Date de dépôt: **18.06.91**

(54) **Matériau composite hémocompatible.**

(30) Priorité: **25.06.90 FR 9007984**

(43) Date de publication de la demande:
**08.01.92 Bulletin 92/02**

(45) Mention de la délivrance du brevet:
**23.08.95 Bulletin 95/34**

(84) Etats contractants désignés:
**AT BE CH DE DK ES FR GB GR IT LI LU NL SE**

(56) Documents cités:
**EP-A- 0 068 509**
**EP-A- 0 100 285**
**FR-A- 2 105 502**

**JOURNAL OF APPLIED POLYMER SCIENCE,
vol. 35, no. 1, janvier 1988, pages 115-125,
John Wiley & Sons, Inc.; H. MIYAMA et al.**

(73) Titulaire: **HOSPAL INDUSTRIE
7, Avenue Lionel Terray,
BP 126
F-69883 Meyzieu Cédex (FR)**

(72) Inventeur: **Dejardin, Philippe
17 rue Vauban
F-67000 Strasbourg (FR)**
Inventeur: **Schmitt, Adrien
5 rue des anémones
F-67450 Mundolsheim (FR)**
Inventeur: **Feng, Yan
7 rue de la Broque
F-67000 Strasbourg (FR)**

Rank Xerox (UK) Business Services
(3.10/3.09/3.3.3)

**Description**

La présente invention concerne le domaine technique des matériaux composites comprenant une membrane support revêtue d'un additif réduisant le caractère thrombogène du support.

Ces matériaux sont largement répandus dans le domaine médical afin d'assurer le traitement de divers liquides biologiques et notamment de sang.

Or,à l'état normal, le sang circule dans les vaisseaux du corps humain ; ces vaisseaux possèdent une surface adaptée à cette fin, c'est-à-dire une surface non-thrombogène au contact de laquelle le système de la coagulation ne s'active pas et à laquelle les plaquettes n'adhèrent pas.

La mise en circulation extracorporelle du sang à des fins de traitement impose un contact entre le sang et une surface artificielle. Ainsi, par exemple dans le cas du traitement du sang par dialyse, le sang entre en contact avec les lignes et avec la membrane de dialyse. Il en est de même pour la plasmaphérèse, ou pour des traitements par contact avec des supports greffés d'enzymes ou de substances destinées à l'épuration.

Mais, le contact du sang avec une surface artificielle entraîne généralement une activation du système de la coagulation et le dépôt des plaquettes sanguines, ce qui risque de conduire à des thromboses, soit localement au contact de la surface artificielle, soit plus généralement à l'intérieur du corps humain.

Les matériaux utilisés pour la circulation extracorporelle et le traitement du sang sont donc étudiés et sélectionnés avec soin pour leur qualité de biocompatibilité et notamment d'hémocompatibilité. Cependant, ce n'est pas toujours suffisant. Aussi, des tentatives d'amélioration de ces supports par différents traitements ont déjà été, effectuées dans l'art antérieur.

Ainsi, l'article de MIYAMA, intitulé "Graft copolymerization of methoxypoly (ethylene glycohol) methacrylate onto polyacrylonitrile and evaluation of nonthrombogenicity of the copolymer", publié dans le "Journal of Applied Polymer Science", volume 35, 115-125 de 1988, décrit un matériau constitué par la copolymérisation greffée de méthacrylate de méthoxypolyéthylène glycol et de polyacrylonitrile thioamide. Les auteurs de cet article ont ainsi mis en évidence l'amélioration du caractère non-thrombogène du copolymère greffé par rapport au polymère d'acrylonitrile.

Cependant, l'obtention d'un tel copolymère nécessite l'utilisation d'un procédé de fabrication particulier. En effet, les techniques décrites dans ce document ne permettent pas, grâce à des opérations relativement simples, d'améliorer le caractère non-thrombogène d'une membrane support, sans modification substantielle de sa nature.

Or, il est parfois intéressant de pouvoir conserver la nature ainsi que les propriétés de certaines membranes support existantes tout en améliorant leur caractère d'hémocompatibilité.

A cette fin, la présente invention propose un matériau composite comprenant une membrane support constituée essentiellement par un copolymère d'acrylonitrile et d'au moins un monomère ionique ou ionisable revêtue d'un additif réduisant le caractère thrombogène du support, ledit additif étant un copolymère dont l'un au moins des monomères comprend le motif polyoxyéthylène avec un degré de polymérisation supérieur ou égal à trois, caractérisé en ce que l'additif est adsorbé sur la membrane support et en ce qu'il comporte au moins l'acrylonitrile comme comonomère.

L'adsorption de l'additif permet de conserver la nature chimique de la membrane support et donc ses propriétés.

Le phénomène d'adsorption fait intervenir des liaisons physiques telles que les liaisons ioniques, les liaisons hydrophiles, les liaisons hydrophobes et les forces de Van der Waals.

L'amélioration de l'adsorption de l'additif sur la membrane support est obtenue grâce aux intéractions existant entre les groupements acrylonitrile de l'additif et ceux du support.

Ainsi, grâce à un procédé de traitement simple, il est possible d'améliorer nettement le caractère non-thrombogène d'un support.

En outre, le traitement par adsorption est économique comparé aux autres traitements tels que le greffage en masse. En effet, on obtient une efficacité maximale pour une quantité minimale d'additif utilisée, car il suffit d'une couche superficielle d'additif pour obtenir l'effet désiré.

Selon une forme particulièrement avantageuse de l'invention, le degré de polymérisation du polyoxyéthylène est égal à 23.

Ainsi, les chaînons de polyoxyéthylène sont suffisamment longs pour provoquer à la surface de la membrane un certain encombrement stérique qui empêche le dépôt des plaquettes et du fibrinogène ; en outre, le caractère hydrophile du polyoxyéthylène conduit à former à la surface du support un environnement aqueux qui contribue à accentuer le caractère de biocompatibilité de la membrane.

Selon un mode de réalisation particulier de la présente invention, l'additif comporte en outre un comonomère chargé positivement.

Cette charge positive, qui peut par exemple provenir d'un ammonium quaternaire, permet d'améliorer l'ancrage entre l'additif et le support grâce à une liaison ionique.

De nombreux avantages de la présente invention apparaîtront à la lecture de la description plus détaillée qui va suivre.

La figure I représente, de façon schématique et sans échelle déterminée, un montage expérimental permettant de mettre en évidence l'amélioration des propriétés de non-thrombogénicité du matériau selon l'invention.

La figure II illustre la réduction de l'adhésion plaquettaire induite par le matériau selon l'invention par rapport à un matériau de l'art antérieur, en absence de globule rouge.

La figure III illustre la réduction de l'adhésion plaquettaire induite par le matériau selon l'invention par rapport à un matériau de l'art antérieur en présence de globules rouges.

Les figures IV, V et VI illustrent les résultats de dosages des taux plasmatiques respectivement du Fibrinopeptide A, de la $\beta$ thromboglobuline et de la protéine activée C3a, lors de tests de dialyse ex-vivo avec des matériaux selon l'invention comparativement avec des matériaux de l'art antérieur.

Les membranes supports selon l'invention sont constituées par un copolymère d'acrylonitrile et d'au moins un monomère ionique ou ionisable, telles qu'elles sont décrites dans la demande FR 2 105 502. Les monomères ioniques ou ionisables sont essentiellement des monomères oléfiniquement insaturés et comportant au moins un groupement fonctionnel tel que le groupement acide sulfonique ou phosphonique. Les groupements acides se trouvent de préférence sous forme de sels, tels que les sels de sodium, de potassium, d'ammonium.

Un exemple de membrane support convenant particulièrment bien est une membrane d'acrylonitrile et de méthallylsulfonate de sodium telle que la membrane de dialyse HOSPAL AN 69 (marque déposée). Une membrane de plasmaphérèse constituée de méthylméthacrylate et d'acrylonitrile est également un exemple de membrane convenant aux fins de la présente invention.

L'additif utilisé selon la présente invention est un polymère dont l'un des comonomères est de l'acrylonitrile et dont au moins l'un des comonomères est un macromonomère de polyoxyéthylène $(CH_2CH_2O)n$. Le degré de polymérisation n du macromère de polyoxyéthylène est une variable supérieure ou égale à trois. En effet, plus le degré de polymérisation sera important, plus la longeur des chaînons sera grande et donc plus l'environnement aqueux que crée l'hydrophilie du polyoxyéthylène représentera une couche épaisse. Or, cet environnement est fortement hémocompatible. En outre, les chaînons de polyoxyéthylène provoquent un encombrement stérique à la surface du support qui empêche le dépôt des plaquettes et l'adsorption du fibrinogène.

Certains auteurs avancent également l'hypothèse selon laquelle ces chaînons flexibles joueraient le rôle de cils vibratiles dont le mouvement empêcherait le dépôt des plaquettes ou du fibrinogène.

Parmi les macromonomères de polyoxyéthylène convenant à la présente invention, on peut citer en particulier le polyacrylate de tri(éthylène glycol), le méthacrylate de nonaéthylène glycol, le méthacrylate de méthoxynona(éthylène glycol), le méthacrylate de méthoxy 23 éthylène glycol.

Selon la présente invention, afin d'améliorer l'ancrage de l'additif au support, on peut lui adjoindre au moins un monomère chargé positivement. Cette charge positive permettra, dans le cas d'un support possédant des sites électronégatifs, tels que les sites sulfonates de la membrane HOSPAL AN 69 (marque déposée), de créer des liaisons ioniques renforçant l'adsorption de l'additif au support. La charge positive peut par exemple être celle d'un ammonium quaternaire comme c'est le cas si on adjoint à l'additif un monomère tel que le chlorure de méthacrylate de triméthyl amino éthyle.

L'adsorption de l'additif sur la membrane support peut être effectuée par circulation lente, au contact de la membrane support, de l'additif dans une solution tampon au pH approprié, puis par rinçage abondant du support avec cette solution tampon.

Ainsi dans le cas où la membrane support se présente sous forme de fibres creuses disposées à l'intérieur d'un hémodialyseur, l'adsorption peut être effectuée par injection lente de l'additif dans un tampon tyrode (NaCl, KCl, NaHCO$_3$, NaH$_2$PO$_4$, H$_2$O) par exemple.

Les qualités que présente le matériau composite de la présente invention, de diminuer l'adsorption du fibrinogène et l'adhésion des plaquettes, peuvent être testées grâce à des méthodes de mesure de l'activité radioactive après marquage du fibrinogène et des plaquettes, par exemple à l'iode 125 et à l'indium 111 respectivement.

Exemple 1 :

On fabrique un additif convenant à la présente invention par copolymérization radicalaire d'acrylonitrile et de monométhacrylate de méthoxy polyéthylène glycol. On appelle A(n) le méthacrylate de polyéthylène glycol dont le degré de polymérisation est n et B l'acrylonitrile.

Du diméthylformamide (DMF), du diméthylsulphoxide (DMSO) et de l'acrylonitrile (B) sont distillés deux fois sur $CaH_2$, sous pression réduite pour ce qui concerne le DMF et le DMSO. On cristallise l'azobis-isobutyronitrile utilisé comme initiateur à partir d'une solution de méthanol toluène.

Les méthacrylates de méthoxy polyéthylène glycol A(5), A(8) et A(21) sont utilisés directement tels qu'ils sont fournis par Polysciences.

Les monomères, le solvant et l'initiateur sont introduits dans un réacteur en verre à double paroi muni d'un agitateur magnétique ou mécanique et relié à un thermostat externe permettant à la température d'être contrôlée à ± O,1 °C.

Le système est ensuite dégazé par 3 cycles successifs de balayage vide-argon, et la réaction est ensuite effectuée à une température constante de 60 °C sous une légère pression d'argon.

La consommation d'acrylonitrile est contrôlée par chromatographie gaz-liquide.

La récupération et la purification des copolymères sont effectuées suivant l'une des procédures suivantes, selon la composition de l'échantillon :

a) les copolymères pauvres en méthacrylate de polyoxyéthylène sont précipités à partir du milieu de réaction dans de l'eau distillée en excès (10 fois), et ils sont ensuite purifiés par précipitation dans de l'eau à partir de leur solution dans du DMF, suivie d'un lavage grossier avec du méthanol dans un mixeur ,

b) les copolymères riches en méthacrylate de polyoxyéthylène sont précipités à partir du milieu réactionnel dans du diéthylether en excès (dix fois) ; ils sont ensuite dissous dans de l'eau distillée et les solutions sont ensuite complètement dialysées pendant au moins 48 h en utilisant des membranes cellulosiques Spectrapor dont le seuil de coupure est de 3000 daltons.

Les copolymères sont ensuite lyophilisés si on veut les conserver.

Exemple 2 :

On fabrique un additif convenant à la présente invention par copolymérisation du mélange réactionnel suivant :

- chlorure de méthacrylate de trimethylaminoéthyle commercial
- acrylonitrile,
- méthacrylate de méthoxypolyéthylène glycol dont le degré de polymérisation est 23.

La copolymérisation est effectuée à 30 °C, en présence de $KClO_3$ et de $NaHSO_3$ en milieu aqueux.

Le tableau suivant retrace pour deux échantillons X et Y, la composition molaire en monomères dans le mélange réactionnel initial (f) et dans le copolymère obtenu (F), ainsi que la composition massique correspondante (W) dans le copolymère.

| ECHANTILLON | | A | B | C |
|---|---|---|---|---|
| X<br>Non soluble<br>dans l'eau | f | 0,025 | 0,028 | 0,947 |
| | F | 0,019 | 0,187 | 0,794 |
| | W | 0,015 | 0,810 | 0,165 |
| Y<br>Soluble dans<br>l'eau | f | 0,033 | 0,050 | 0,917 |
| | F | 0,026 | 0,228 | 0,746 |
| | W | 0,018 | 0,840 | 0,132 |

Monomère A : Chlorure de méthacrylate de triméthylaminoéthyle

Monomère B : macromère de polyéthylène glycol (MG 23)

Monomère C : acrylonitrile.

Exemple 3 :

On utilise comme membrane-support des fibres constituées par un copolymère d'acrylonitrile et de méthallyl sulfonate de sodium : HOSPAL AN 69 (marque déposée).

Ces fibres, au nombre de 48, sont insérées dans un minimodule d'hémodialyse. Le diamètre interne des fibres est de 270 $\mu$m et leur longueur est d'environ 20cm.

On utilise comme additif le terpolymère Y obtenu dans l'exemple 2. Ce terpolymère Y est en solution dans du tampon TS (Tyrode Simple) dont la composition pour 1l est la suivante :
- NaCl : 8 g
- KCl : 0,2 g
- NaHCO$_3$ : 1 g
- NaH$_2$PO$_4$-H$_2$O : 0,06 g

Afin d'obtenir le matériau composite de la présente invention, on traite par adsorption la surface interne des fibres creuses en injectant lentement à l'intérieur du mini-module une solution de terpolymère Y dans le tampon TS, et en rinçant ensuite abondamment avec ce tampon, dont le pH est 7,5.

Les paramètres de cette adsorption sont les suivants :
- concentration du terpolymère en solution : 5 g/l
- volume de solution injectée : 25 ml
- débit de l'injection : 0,1 ml/min
- température ambiante.

Exemple 4 :

On teste l'adsorption du fibrinogène sur un minimodule obtenu selon l'exemple 3.

A cette fin, une solution de fibrinogène est marquée à l'Iode 125 selon la technique à l'iodogène.

La cinétique d'adsorption du fibrinogène est effectuée grâce au montage expérimental représenté sur la figure I.

Deux pousse-seringues P1 et P2, contenant l'un, la solution, l'autre, le solvant, sont pilotés par un microordinateur M1. Le débit total, la concentration de la solution injectée, la durée et le nombre de passages de la solution peuvent être ainsi programmés. Relié par un tube en teflon aux deux pousse-seringues, le minimodule M est placé dans une fente d'un détecteur (cristal de NaI), qui est connecté à une échelle de comptage Ech reliée également au microordinateur M1. A la sortie du module M, la solution est dirigée d'abord vers une cellule UV pour le contrôle de la concentration en protéines ; elle est ensuite recueillie dans un récipient posé sur une balance électronique BL dont les données sont acquises par le microordinateur M1. L'ensemble des données cinétiques instantanées est visualisé sur l'écran et enregistré dans un fichier, lequel peut être utilisé pour un traitement ultérieur.

La thermostation du minimodule à 37°C est effectuée par circulation du tampon dans le compartiment du dialysat. Le débit de ce tampon est contrôlé par un débitmètre à flotteur.

L'introduction de la solution de fibrinogène dans les minimodules se fait par le déplacement du tampon pour éviter la formation d'une interface air-solution. Le passage de la solution ou suspension radioactive est encadrée par deux passages de tampon de même durée.

La durée de passage de la solution et celle de chaque rinçage par le tampon sont égales à 25 min. Le débit est fixé à 2 ml/min.

Aprés l'expérience, les faisceaux de fibres sont découpés en morceaux de 2 cm pour déterminer la concentration en surface en fonction de la position sur le faisceau.

Afin de montrer l'efficacité de l'additif en ce qui concerne la réduction de l'adsorption du fibrinogène, la même expérience est effectuée sur un minimodule de fibres creuses constituées par le matériau composite selon la présente invention (obtenu selon l'exemple 3) et un minimodule de fibres creuses constituées par la membrane HOSPAL AN 69 (marque déposée).

Le tableau suivant regroupe des résultats obtenus pour 3 séries d'essais comparatifs, effectués à partir de solutions de concentrations en fibrinogène différentes.

| MODULE | Concentration fibrinogène g/l | $\gamma$ (S$^{-1}$) | Pente (CPM/s) | k (cm/s) | $\Gamma$ g/cm$^2$ | R |
|---|---|---|---|---|---|---|
| A | 0,0055 | 370 | 159,5 | 101,5 | 0,67 | 0 |
| A traité | 0,0063 | 370 | 17,3 | 11,0 | 0,085 | 88,0 |
| B | 0,0050 | 518 | 139 | 105,5 | 0,73 | 0 |
| B traité | 0,0047 | 522 | 7,36 | 4,0 | 0,019 | 97,0 |
| C | 0,339 | 505 | 563 | 9,89 | 4,86 | 0 |
| C traité | 0,316 | 505 | 133 | 3,07 | 0,618 | 87,3 |

$\gamma$ exprime le taux de cisaillement.

$\Gamma$ représente la concentration interfaciale superficielle du fibrinogène à un temps t.

k est la constante d'adsorption apparente du fibrinogène, qui permet de comparer les résultats en s'affranchissant de la concentration en fibrinogène de la solution.

La pente, exprimée en CPM/s (coup par minute par seconde) est représentative de la cinétique du phénomène d'adsorption.

Dans ces trois séries d'expériences, on observe que les minimodules contenant un matériau selon l'invention adsorbent nettement moins de fibrinogène que les témoins, la réduction variant entre 87 et 97 %.

Exemple 5 :

On étudie la cinétique d'adhésion des plaquettes comparativement avec un minimodule de fibres creuses constituées par du matériau composite selon la présente invention et avec un minimodule de fibres creuses en membrane HOSPAL AN 69 (marque déposée) servant de témoin. Le montage expérimental est celui de l'exemple précédent.

Les plaquettes sont marquées à l'indium 111 dans le tampon TA (Tyrode Albumine 0,35 %). Le temps de passage de la suspension plaquettaire est variable (5 ou 10 min) ; en revanche, le temps de chaque rinçage est fixé à 3 min.

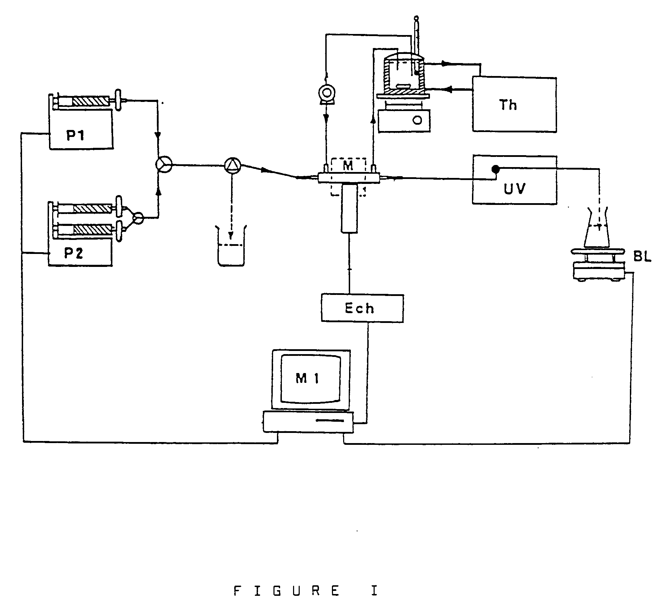

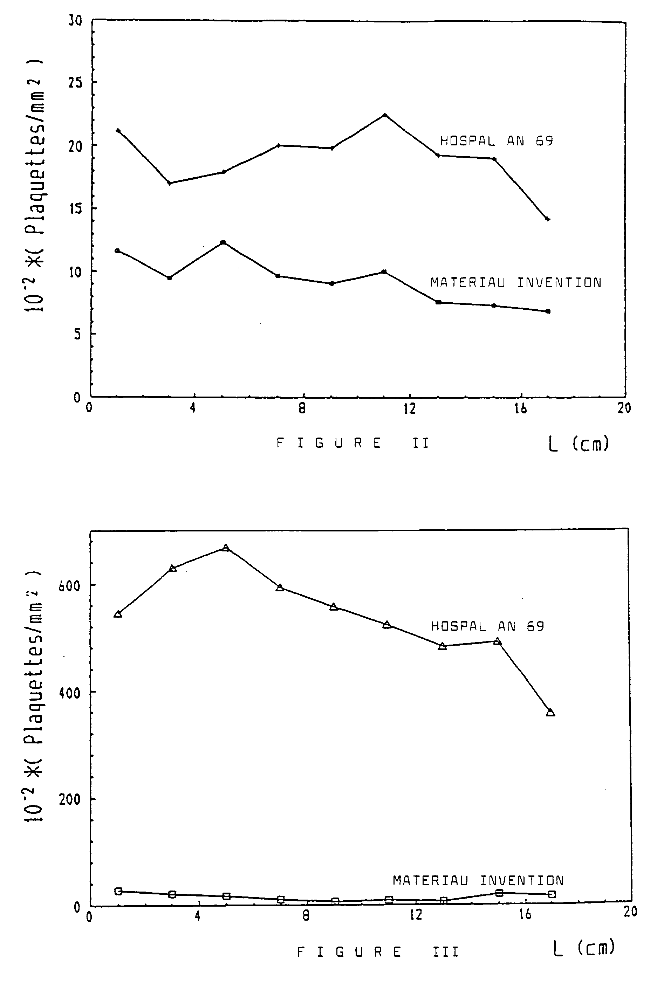

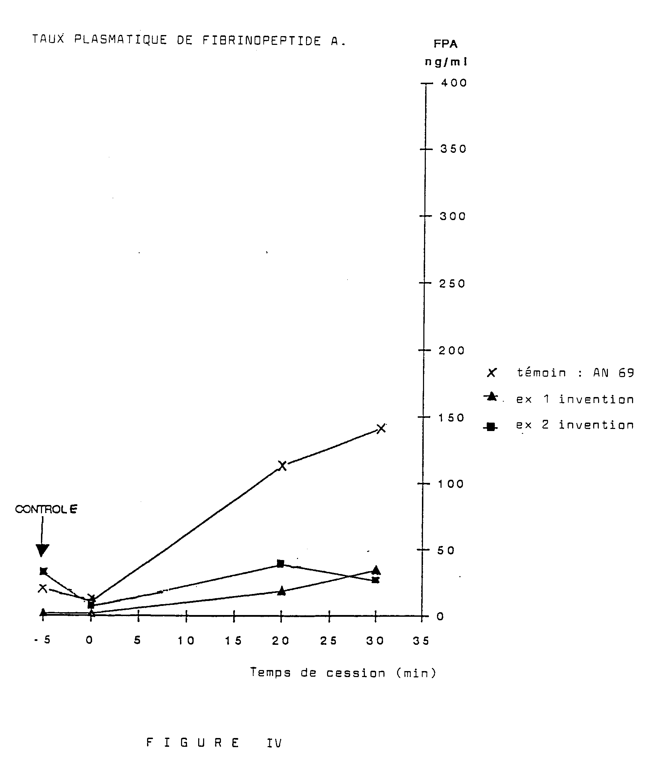

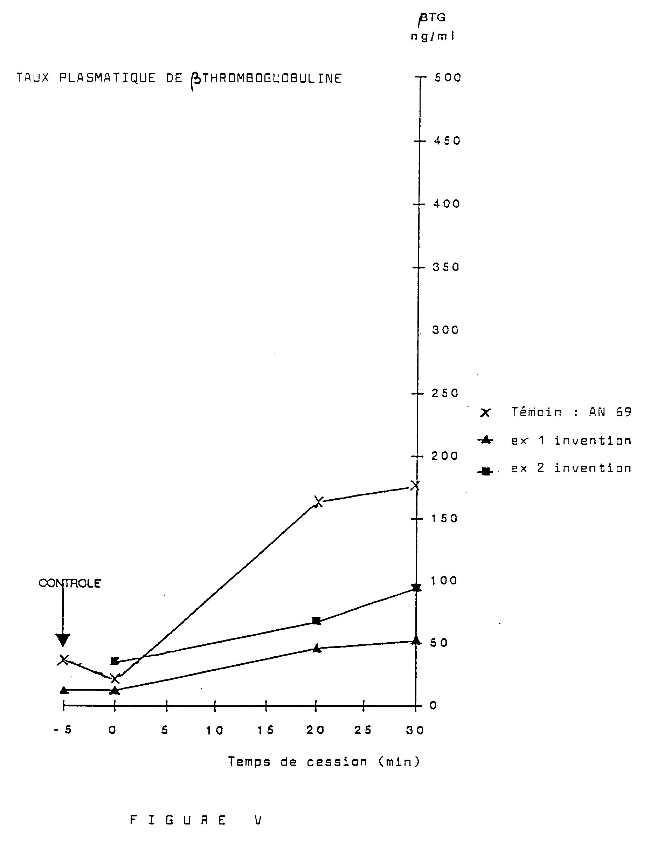

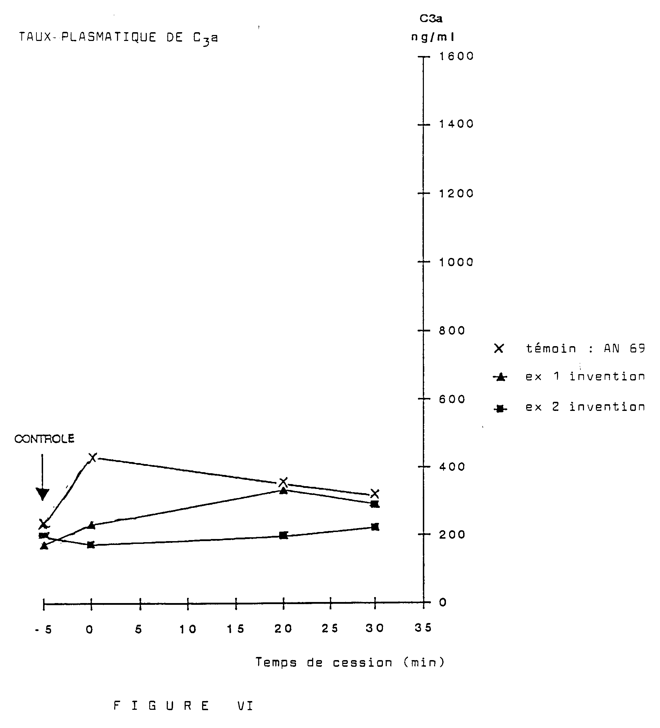

Les courbes de la figure 2 illustrent l'amélioration du caractère non-thrombogène du matériau selon l'invention par rapport à la membrane témoin.

Ces courbes représentent la concentration en plaquettes en surface en fonction de la distance par rapport à l'entrée du minimodule. La concentration en plaquettes de la solution utilisée est de 300 000 pl/$\mu$l, la durée du contact entre la surface des fibres et la solution de plaquettes est de 10 minutes ; le taux de cisaillement est de 500 s$^{-1}$, la réaction est effectuée à 37°C. La solution de plaquettes est dépourvue de globule rouge.

Cette figure montre, qu'en absence de globule rouge, l'adhésion plaquettaire est faible, mais que le matériau composite selon l'invention produit une adhésion des plaquettes inférieure de 40 à 50 % à l'adhésion des plaquettes remarquée sur les fibres témoins.

Exemple 6 :

Les conditions d'expérimentation sont les mêmes que dans l'exemple précédent.

Cependant, les plaquettes sont mises en solution dans une solution contenant 40 % de globules rouges, ce qui correspond assez bien à la concentration du sang pour un hématocrite normal. La concentration en plaquettes est alors de 180 000 pl/$\mu$l.

Le taux de cisaillement est de 500 s$^{-1}$ et la réaction est effectuée à température ambiante.

Les résultats obtenus sont illustrés sur la figure 3 qui représente, pour chacun des minimodules (selon l'invention et selon le module-témoin) l'activité de la surface, en fonction du temps.

Dans ces conditions encore, l'adhésion des plaquettes est nettement moindre en utilisant le matériau composite selon l'invention, qu'en utilisant les fibres creuses témoins.

Exemple 7 :

On évalue la biocompatibilité du matériau objet de l'invention grâce aux mesures des taux de Fibrinopeptide A, de $\beta$ thromboglobuline et de la protéine C3a au cours de séances de dialyse ex-vivo.

A cette fin, on utilise des mini-dialyseurs de fibres creuses dont les caractéristiques sont les suivantes :
- membrane HOSPAL AN 69 (marque déposée)
- diamètre interne des fibres : 240 $\mu$m
- diamètre externe des fibres : 340 $\mu$m
- nombre de fibres : 340
- surface interne : 512 cm$^2$

Après rinçage des mini-dialyseurs avec une solution aqueuse de NaCl à 0,9 g/l, deux d'entre eux sont traités par adsorption de l'additif Y obtenu selon l'exemple 2. Un troisième est conservé intact pour servir de témoin. Le traitement par adsorption est effectué grâce au passage dans les fibres d'une solution de polymère à 5 g/l dans un tampon tyrode simple.

On fait passer 5 ml à un débit de 6 ml/min puis 17 ml à un débit de 0,1 ml/min.

Après traitement, les dialyseurs sont rincés par une solution aqueuse de NaCl à 0,9 g/l, héparinée à 2,5 UI/ml pour le compartiment sang.

Pour effectuer les tests ex-vivo, on maintient le liquide de rinçage dans le compartiment dialysat en fermant l'entrée et la sortie du liquide de dialyse et on fait passer le sang d'un donneur dans le compartiment sang à un débit de 10 ml/min, pendant 34 min. Le taux de cisaillement est de 360 s$^{-1}$. Après son passage dans l'hémodialyseur, le sang n'est pas restitué au patient.

On effectue alors le dosage d'un certain nombre de substances plasmatiques qui permettent d'évaluer la biocompatibilité du matériau.

La figure IV représente le taux plasmatique de Fibrinopeptide A de deux essais réalisés avec le matériau de l'invention ainsi que le taux du témoin constitué par une membrane HOSPAL AN 69 (marque déposée) sans additif.

Le Fibrinopeptide A est un indicateur précoce d'une activation du phénomène de la coagulation.

Les résultats représentés sur cette figure montrent bien l'amélioration du caractère non-thrombogène du matériau objet de l'invention.

La figure V illustre, de la même façon, les taux plasmatiques de $\beta$ thromboglobuline qui est un marqueur de l'activation plaquettaire.

Ici encore, on remarque le très bon comportement du matériau objet de l'invention par rapport à l'art antérieur.

La figure VI représente les résultats du dosage plasmatique de la protéine activée C3a dont la présence indique une activation du système du complément.

Les résultats obtenus avec le matériau de l'invention sont sensiblement égaux à ceux obtenus avec la membrane HOSPAL AN 69 (marque déposée) qui est déjà réputée pour ses performances sur ce point. On observe donc un maintien de la bonne qualité du support lors de l'adsorption de l'additif Y.

Exemple 8 :

On vérifie le maintien des performances (dialyse et perméabilité) du matériau de l'invention par rapport au support non revêtu de l'additif.

On utilise à cette fin un minidialyseur équipé de 170 fibres en membrane HOSPAL AN 69 (marque déposée).

On effectue sur ce dialyseur les mesures suivantes :
- Ultrafiltration
- Clairance Urée
- Clairance Vitamine B 12.

Après avoir effectué ces mesures, on traite le dialyseur par adsorption du terpolymère Y de la même façon que dans l'exemple précédent. On obtient ainsi un matériau selon la présente invention.

On mesure à nouveau l'ultrafiltration ainsi que les clairances de l'urée et de la vitamine B12.

Les mesures d'ultrafiltration sont effectuées au bain de dialyse sous une pression transmembranaire de 200 mm Hg.

Les mesures de clairance effectuées sont corrigées pour être ramenées aux mêmes conditions de débit à l'entrée du compartiment sang (soit 3,5 ml/min) et du compartiment dialysat (soit 10,5 ml/min), le débit d'ultrafiltration étant nul.

Les résultats avant et après traitement sont regroupés dans le tableau suivant :

|  | Ultrafiltration en ml/H.m².mmHg | Clairance Urée | Clairance Vit B 12 |
|---|---|---|---|
| Avant traitement | 52 | 3,1 | 1,5 |
| Après traitement = invention | 48 | 3,1 | 1,5 |

Les résultats montrent que les performances du matériau sont maintenues lorsqu'il est revêtu par adsorption de l'additif Y. (La baisse apparente de l'ultrafiltration est certainement dûe aux conditions de la mesure qui n'a pu être effectuée qu'après 2 jours).

## Revendications

1. Matériau composite comprenant une membrane support constituée essentiellement par un copolymère d'acrylonitrile et d'au moins un monomère ionique ou ionisable revêtue d'un additif réduisant le caractère thrombogène du support, ledit additif étant un copolymère dont l'un au moins des monomères comprend le motif polyoxyéthylène avec un degré de polymérisation supérieur ou égal à trois, caractérisé en ce que l'additif est adsorbé sur la membrane support et en ce qu'il comporte au moins l'acrylonitrile comme comonomère.

2. Matériau selon la revendication 1, caractérisé en ce que l'un des comonomères de l'additif comprenant le motif polyoxyéthylène est du méthacrylate de méthoxypolyéthylène glycol.

3. Matériau selon l'une des revendications 1 et 2, caractérisé en ce que le degré de polymérisation du polyoxyéthylène est égal à 23.

4. Matériau selon l'une des revendications précédentes, caractérisé en ce que l'additif comporte en outre un comonomère chargé positivement.

5. Matériau selon la revendication 4, caractérisé en ce que le comonomère chargé positivement est un ammonium quaternaire.

6. Matériau selon l'une des revendications 4 et 5,
caractérisé en ce que le comonomère chargé positivement est un halogénure de méthacrylate de triméthylaminoéthyle.

7. Matériau selon l'une des revendications 4 à 6,
caractérisé en ce que l'additif est constitué par :
   - 2,5 % en mole de comonomère chargé positivement,
   - 23 % en mole de méthacrylate de méthoxypolyéthylène glycol,
   - 74,5 % en mole d'acrylonitrile.

8. Matériau selon l'une des revendications précédentes,
caractérisé en ce que la membrane support est constituée par un copolymère d'acrylonitrile et de méthallyl sulfonate de sodium.

9. Utilisation du matériau selon l'une des revendications précédentes, pour la fabrication d'une membrane de dialyse du sang.

**Claims**

1. Composite material comprising a support membrane which consists essentially of a copolymer of acrylonitrile and at least one ionic or ionisable monomer and is coated with an additive reducing the thrombogenic character of the support, the said additive being a copolymer of which at least one of the monomers comprises the polyoxyethylene unit with a degree of polymerisation greater than or equal to three, characterised in that the additive is adsorbed on the support membrane and in that it comprises at least acrylonitrile as comonomer.

2. Material according to Claim 1, characterised in that one of the comonomers of the additive comprising the polyoxyethylene unit is methoxypolyethylene glycol methacrylate.

3. Material according to one of Claims 1 and 2, characterised in that the degree of polymerisation of the polyoxyethylene is equal to 23.

4. Material according to one of the preceding claims, characterised in that the additive additionally comprises a positively charged comonomer.

5. Material according to Claim 4, characterised in that the positively charged comonomer is a quaternary ammonium.

6. Material according to one of Claims 4 and 5, characterised in that the positively charged comonomer is a trimethylaminoethyl methacrylate halide.

7. Material according to one of Claims 4 to 6, characterised in that the additive consists of:
   - 2,5 mol % of positively charged comonomer,
   - 23 mol % of methoxypolyethylene glycol methacrylate and
   - 74,5 mol % of acrylonitrile.

8. Material according to one of the preceding claims, characterised in that the support membrane consists of a copolymer of acrylonitrile and sodium methallylsulphonate.

9. Use of the material according to one of the preceding claims, for the production of a blood dialysis membrane.

**Patentansprüche**

1. Verbundmaterial mit einem Membranträger, der im wesentlichen aus einem Acrylonitril-Copolymer und zumindest einem ionischen oder ionisierbaren Monomer besteht, wobei der Membranträger mit einem Additiv beschichtet ist, das die thrombogene Eigenschaft des Trägers reduziert, wobei das Additiv ein Copolymer ist, von dem zumindest eines der Monomere die Grundeinheit Polyoxyethylen mit einem Polymerisationsgrad umfaßt, der größer oder gleich als 3 ist, dadurch gekennzeichnet, daß das Additiv auf den Membranträger adsorbiert ist, und daß es zumindest Acrylonitril als Comonomer umfaßt.

2. Material nach Anspruch 1, dadurch gekennzeichnet, daß das eine der Comonomeren des Additivs, das die Grundeinheit Polyoxyethylen umfaßt, Methoxypolyethylenglykolmethacrylat ist.

3. Material nach Anspruch 1 oder 2, dadurch gekennzeichnet, daß der Polymerisationsgrad von Polyoxyethylen 23 ist.

4. Material nach einem der vorangehenden Ansprüche, dadurch gekennzeichnet, daß das Additiv zusätzlich ein positiv aufgeladenes Comonomer umfaßt.

5. Material nach Anspruch 4, dadurch gekennzeichnet, daß das positiv aufgeladene Comonomer quaternäres Ammonium ist.

6. Material nach einem der Ansprüche 4 und 5, dadurch gekennzeichnet, daß das positiv aufgeladene Comonomer Trimethylaminoethylmethacrylathalogenid ist.

7. Material nach einem der Ansprüche 4 bis 6, dadurch gekennzeichnet, daß das Additiv gebildet ist durch :
   - 2,5 Mol% positiv geladenes Comonomer,
   - 23 Mol% Methoxypolyethylenglykolmethacrylat,
   - 74,5 Mol% Acrylonitril.

8. Material nach einem der vorangehenden Ansprüche, dadurch gekennzeichnet, daß der Membranträger aus Acrylonitril-Copolymer und Natriummethallylsulfonat besteht.

9. Verwendung des Materials nach einem der vorangehenden Ansprüche zur Herstellung einer Blutdialysemembran.

EP 0 465 380 B1

FIGURE I

F I G U R E   II

F I G U R E   III

TAUX PLASMATIQUE DE FIBRINOPEPTIDE A.

FIGURE IV

TAUX PLASMATIQUE DE $\beta$THROMBOGLOBULINE

$\beta$TG
ng/ml

Témoin : AN 69
ex 1 invention
ex 2 invention

CONTROLE

Temps de cession (min)

F I G U R E   V

TAUX-PLASMATIQUE DE $C_3a$

F I G U R E   VI